# EUROPEAN PATENT APPLICATION

(11) **EP 4 293 100 A1**
(43) Date of publication of application: **20.12.2023**
(21) Application number: 21925650.0
(22) Date of filing: 12.02.2021
(51) Int. Cl.: C12M 1/00

(54) **ORGAN-ON-CHIP AND BIOLOGICAL FUNCTION REPRODUCTION METHOD**

(71) Applicant: Enplas Corporation, Kawaguchi-shi, Saitama 332-0034 (JP)
(72) Inventor: ONO, Koichi, Kawaguchi-shi, Saitama 332-0034 (JP); WATANABE, Yasuhiro, Kawaguchi-shi, Saitama 332-0034 (JP)
(74) Representative: J A Kemp LLP
(86) International application number: PCT/JP2021/005258
(87) International publication number: WO 2022/172397

(57) **Abstract**

An object is to provide a novel organ-on-a-chip for forming a blood vessel model. An organ-on-a-chip 100 of the present invention is a chip for reproducing a biological process between a cell mass and a blood vessel. The organ-on-a-chip 100 includes a first channel 10, a second channel 20, and a third channel group. The first channel 10 is an introduction channel for introducing vascular endothelial cells for forming a blood vessel model on the inner wall of the first channel 10 through cell culture. The second channel 20 is an introduction channel for introducing a sample containing a cell mass from an upstream side toward a downstream side, and includes a trap portion 22 for trapping the cell mass and located between the upstream side and the downstream side inside the second channel 20. The third channel group includes a plurality of third channels 30, and the third channels 30 are channels that communicate between the first channel 10 and the second channel 20.

## Description

### Technical Field

The present invention relates to an organ-on-a-chip for reproducing a biological process between a cell mass and a blood vessel, and a method for reproducing a biological function using the organ-on-a-chip.

### Background Art

In recent years, organs-on-chips that reproduce biological functions on chips have been attracting attention. One of the organs to be reproduced on a chip is a blood vessel. As an example of the organ-on-a-chip that reproduces a blood vessel, a form in which a first channel and a second flow channel are coupled to each other via a third channel is reported (Non-Patent Document 1). For example, with this organ-on-a-chip, an in-vivo reaction can be reproduced as a response of a blood vessel model to a substance released from cancer cells by forming a blood vessel model in the first channel through 3D culture, filling the second channel with cancer cells, and then culturing the cancer cells.

### Citation List

### Non-Patent Document

Non-Patent Document 1: Ioannis K. Zervantonakis et al., PNAS, August 21, 2012, vol. 109, no. 34, 13515-13520

### Summary of Invention

### Technical Problem

However, although the second channel is filled with cancer cells as described above in the above-mentioned organ-on-a-chip in which a blood vessel model is formed, cancer cells can hardly be considered to exist independently of one another in an actual living organism. Accordingly, a technique that achieves a state closer to the in-vivo state in a chip is required.

Therefore, it is an object of the present invention to provide a novel organ-on-a-chip for forming a blood vessel model, for example.

### Solution to Problem

To achieve the above-mentioned object, an organ-on-a-chip of the present invention is a chip for reproducing a biological process between a cell mass and a blood vessel, the chip including:
a first channel;
a second channel; and
a third channel group,
wherein the first channel is an introduction channel for introducing vascular endothelial cells for forming a blood vessel model on an inner wall of the first channel through cell culture,
the second channel is an introduction channel for introducing a sample containing a cell mass from an upstream side toward a downstream side, and includes a trap portion for trapping the cell mass and located between the upstream side and the downstream side inside the second channel, and
the third channel group includes a plurality of third channels, and the third channels are channels that communicate between the first channel and the second channel.

A biological function reproduction method of the present invention uses the above-mentioned organ-on-a-chip, and includes:
performing first culturing for forming a blood vessel model on an inner wall of the first channel by introducing vascular endothelial cells to the first channel and culturing the cells; and
performing second culturing for introducing a sample containing a cell mass to the second channel from an upstream side of the second channel and culturing the cell mass in a state in which the cell mass is trapped in the trap portion in the second channel.

### Advantageous Effects of Invention

The inventors of the present invention found that use of a cell mass rather than cells that exist independently of one another is suitable for reproducing a state closer to the in-vivo state in an organ-on-a-chip for forming a blood vessel model. However, the structure of the above-described organ-on-a-chip is based on the premise that the second channel is filled with many free cells, and therefore, even if this organ-on-a-chip is used, it is difficult to generate, for example, an influence of a single cell mass on a blood vessel model under conditions close to the in-vivo conditions. Accordingly, as a result of extensive research, the present inventors found that conditions closer to the in-vivo conditions can be mimicked in the above-mentioned organ-on-a-chip by trapping the cell mass at a desired position in the second channel. With the present invention, the second channel includes the trap portion, and therefore, the cell mass can be trapped in the trap portion by being introduced as a sample, and thus it can be said that conditions closer to the in-vivo conditions can be reproduced.

### Brief Description of Drawings

[FIG. 1] FIG. 1 shows schematic diagrams illustrating an example of an organ-on-a-chip of Embodiment 1.
[FIG. 2] FIG. 2 is a partial plan view of an example of an organ-on-a-chip of Embodiment 1.
[FIG. 3] FIG. 3 shows schematic diagrams illustrating an example of a portion of an organ-on-a-chip of Embodiment 2.
[FIG. 4] FIG. 4 shows schematic diagrams illustrating an example of a portion of an organ-on-a-chip of Embodiment 2.
[FIG. 5] FIG. 5 shows schematic diagrams illustrating an example of an organ-on-a-chip of Embodiment 3.

### Description of Embodiments

In the organ-on-a-chip of the present invention, the trap portion in the second channel traps the cell mass and allows single cells to pass through, for example.

In the organ-on-a-chip of the present invention, for example, in a direction perpendicular to an axial direction of the second channel, a cross-sectional area of an inner space of the trap portion is smaller than the minimum cross-sectional area of cross sections of the cell mass passing through the center of the cell mass.

In the organ-on-a-chip of the present invention, for example, in the direction perpendicular to the axial direction of the second channel, a cross-sectional area of an inner space of the trap portion is larger than the maximum cross-sectional area of single cells.

In the organ-on-a-chip of the present invention, for example, in the direction perpendicular to the axial direction of the second channel, when an inner space has a circular cross section, a cross section of the trap portion has a diameter of 10 to 300 pm, a cross section of a portion located upstream of the trap portion has a diameter of 200 to 1000 pm, and the diameter of the trap portion is 0.1 to 0.9 times as large as the diameter of the portion located upstream of the trap portion, and when an inner space has a quadrilateral cross section, a cross section of the trap portion has a width and a height of 10 to 300 pm, a cross section of a portion located upstream of the trap portion has a width and a height of 200 to 1000 pm, and the width and the height of the trap portion are 0.1 to 0.9 times as large as the width and the height of the portion located upstream of the trap portion.

In the organ-on-a-chip of the present invention, for example, in the direction perpendicular to the axial direction of the second channel, the trap portion in the second channel has a columnar body that prevents the cell mass from moving toward the downstream side.

In the organ-on-a-chip of the present invention, for example, in the direction perpendicular to the axial direction of the second channel, a height of the columnar body of the trap portion is shorter than a height of the inner space of the trap portion.

In the organ-on-a-chip of the present invention, for example, in the direction perpendicular to the axial direction of the second channel, the inner space of the trap portion has a quadrilateral cross section, and lengths of at least a pair of sides of two pairs of opposite sides of the cross section are smaller than a diameter of the cell mass.

In the organ-on-a-chip of the present invention, for example, a height of the columnar body of the trap portion is shorter than or equal to half of a height of the inner space of the trap portion.

In the organ-on-a-chip of the present invention, for example, the columnar body is disposed at a position 100 to 1000 pm away from a portion of the second channel that communicates with the third channel group.

In the organ-on-a-chip of the present invention, for example, the third channel group is disposed upstream of the trap portion in the second channel.

In the organ-on-a-chip of the present invention, for example, the third channel group is disposed at a position 0 to 1000 pm away from an end on an upstream side of the trap portion in the second channel.

In the organ-on-a-chip of the present invention, for example, in a direction perpendicular to an axial direction of the first channel, a length of an inner circumference of the first channel corresponds to a length of an outer circumference of a blood vessel.

In the organ-on-a-chip of the present invention, for example, in the direction perpendicular to the axial direction of the first channel, when an inner space of the first channel has a circular cross section, the cross section has a diameter of 100 to 500 pm, and when an inner space of the first channel has a quadrilateral cross section, the cross section has a width and a height of 100 to 500 µm.

In the organ-on-a-chip of the present invention, for example, the inner wall of the first channel has a coating layer with an affinity for vascular endothelial cells.

In the organ-on-a-chip of the present invention, for example, when an inner space of each of the third channels has a circular cross section in a direction perpendicular to an axial direction of the third channel, the cross section has a diameter of 10 to 100 pm, and when an inner space of each of the third channels has a quadrilateral cross section in the direction perpendicular to the axial direction of the third channel, the cross section has a width and a height of 10 to 100 pm.

For example, the organ-on-a-chip of the present invention includes a pair of substrates, and one of the substrates is placed on the other substrate. An opposed surface of the one substrate is provided with recessed portions serving as inner spaces of the first channel, the second channel, and the third channel group, and the recessed portions on the opposed surface of the one substrate and an opposed surface of the other substrate form the first channel, the second channel, and the third channel group.

For example, the organ-on-a-chip of the present invention includes a pair of substrates. Opposed surfaces of one of the substrates and the other substrate are each provided with recessed portions serving as inner spaces of the first channel, the second channel, and the third channel group, and the recessed portions on the opposed surface of the first substrate and the recessed portions on the opposed surface of the second substrate form the first channel, the second channel, and the third channel group.

In the biological function reproduction method of the present invention, for example, the vascular endothelial cells are HUVEC cells.

In the biological function reproduction method of the present invention, for example, the vascular endothelial cells are introduced together with a culture medium in the first culturing.

In the biological function reproduction method of the present invention, for example, the cell mass is a cell mass of cancer cells.

In the biological function reproduction method of the present invention, for example, the sample in the second culturing contains the cell mass and a culture medium.

For example, the biological function reproduction method of the present invention further includes performing coating for forming a coating layer with an affinity for vascular endothelial cells on an inner wall of the first channel prior to the first culturing.

In the biological function reproduction method of the present invention, for example, in the coating, a coating liquid that contains a coating agent for fixing the vascular endothelial cells and a solvent that does not contain the coating agent are introduced to the first channel and the second channel, respectively, with a flow rate balance that prevents flow in the third channels. In the first culturing, a culture medium that contains the vascular endothelial cells and a solvent that does not contain the cells are introduced to the first channel and the second channel, respectively, with a flow rate balance that prevents flow in the third channels.

Specifically, the organ-on-a-chip of the present invention may be in a state in which a blood vessel model has not been formed in the first channel yet or in a state in which a blood vessel model has already been formed in the first channel.

Specific examples of the organ-on-a-chip of the present invention and a method using the same, namely the biological function reproduction method of the present invention, will be described with reference to the drawings. In the diagrams, the same reference numeral denotes the same portion. The present invention is in no way limited or restricted to the following embodiments. The description of each embodiment can be applied to the other embodiments unless otherwise stated. In the embodiments, examples of the sizes of portions are shown, but the sizes can be changed as appropriate depending on, for example, the type of vascular endothelial cells, the target size of a blood vessel model formed, the type and the size of a cell mass, and the like.

### (Embodiment 1)

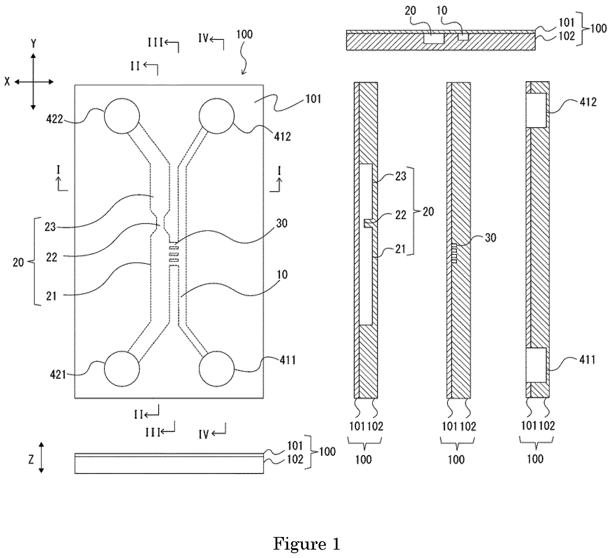

FIG. 1 shows an example of an organ-on-a-chip of this embodiment. FIG. 1 shows schematic diagrams of an organ-on-a-chip 100. The left upper diagram is a top view, the left lower diagram is a side view, the right upper diagram is a cross-sectional view taken along line I-I, and the three right lower diagrams are a cross-sectional view taken along line II-II, a cross-sectional view taken along line III-III, and a cross-sectional view taken along line IV-IV in the order from left to right.

The organ-on-a-chip 100 includes a first channel 10, a second channel 20, and a plurality of third channels 30 (third channel group), which are hollow, and inlets 411 and 421 and outlets 412 and 422, which are openings. An end on the upstream side and an end on the downstream side of the first channel 10 communicate with the inlet 411 and the outlet 412, respectively, and an end on the upstream side and an end on the downstream side of the second channel 20 communicate with the inlet 421 and the outlet 422, respectively.

The organ-on-a-chip 100 is a laminate formed by laminating an upper substrate 101 and a lower substrate 102. Recessed portions on an opposed surface of the lower substrate 102, together with an opposed surface of the upper substrate, form voids (inner spaces) of the first channel 10, the second channel 20, and the third channels 30. In the organ-on-a-chip 100, the inlets 411 and 412 are formed by the recessed portions on the opposed surface of the lower substrate 102 and through holes in the upper substrate 101 located at positions corresponding to the above-mentioned recessed portions, and the outlets 421 and 422 are formed by the recessed portions on the opposed surface of the lower substrate 102 and through holes in the upper substrate 101 located at positions corresponding to the above-mentioned recessed portions.

For example, as described later, a biological process between a blood vessel and a cell mass can be reproduced in the organ-on-a-chip 100 by forming a blood vessel model in the first channel 10 and introducing a sample containing a cell mass to the second channel 20, in the organ-on-a-chip 100. Specifically, the third channels 30 communicate between the first channel 10 in which a blood vessel model is to be formed and the second channel 20 to which the sample is to be introduced. This makes it possible to reproduce, in the organ-on-a-chip 100, reactions such as where the blood vessel model detects a substance derived from the cell mass, and sprouts (buds for vascular branching) from the blood vessel model extend toward the second channel 20 through the third channels 30 (among other reactions), for example. Note that the blood vessel model in the present invention is, for example, a 3D structure formed in a tubular shape through culture of the vascular endothelial cells. For example, vascular pericytes, which are constructional elements of a blood vessel, and the like may be used together with the vascular endothelial cells to form the blood vessel model.

There is no particular limitation on the overall size of the organ-on-a-chip 100. For example, in FIG. 1, the width in the X direction is 10 to 100 mm, the length in the Y direction is 10 to 100 mm, and the thickness in the Z direction is 0.5 to 3 mm.

In this embodiment, the axial direction of the first channel 10 is a direction in which a liquid introduced to the first channel 10 flows, the axial direction of the second channel 20 is a direction in which a liquid introduced to the second channel 20 flows, and the axial direction of each third channel 30 is a direction in which a liquid introduced to the third channel 30 flows.

The first channel 10 is an introduction channel for introducing vascular endothelial cells for forming a blood vessel model on the inner wall of the first channel 10 through cell culture.

There is no particular limitation on the shape and the size of the inner space of the first channel 10. For example, the shape and the size are determined such that the inner wall of the first channel can be used as a scaffold to form a blood vessel model that is equivalent or similar to a blood vessel in vivo. It is preferable that, in a direction perpendicular to the axial direction of the first channel 10, the shape and the size of the inner circumference (a cross section of the inner space) of the first channel 10 are the same as or similar to the shape and the size of the outer circumference of a blood vessel.

In a specific example, in terms of the shape of the first channel 10, the inner space has, for example, a circular cross section in a direction perpendicular to the axial direction of the first channel 10, and the cross section has, for example, a diameter of 100 to 500 pm, 150 to 400 pm, or 200 to 300 pm. The circular cross section may have, for example, a perfect circular shape or an elliptic shape. Also, in terms of the shape of the first channel 10, the inner space may have, for example, a quadrilateral cross section in a direction perpendicular to the axial direction of the first channel, and the cross section has, for example, a width and a height of 100 to 500 pm, 150 to 400 pm, or 200 to 300 pm. The quadrilateral cross section may have, for example, a square shape or a rectangular shape. Moreover, the cross section may have a polygonal shape other than a quadrilateral shape.

There is no particular limitation on the length of the first channel 10 in the axial direction. For example, the length from the upstream end to the downstream end is 0.5 to 80 mm.

When the organ-on-a-chip 100 is used, a blood vessel model is formed on the inner wall of the first channel 10 by introducing the vascular endothelial cells to the first channel 10 and culturing the cells as described later. Accordingly, the inner wall of the first channel 10 may have an affinity for the vascular endothelial cells for the purpose of, for example, further facilitating fixing of the vascular endothelial cells, and specifically, the inner wall may have, for example, a coating layer with an affinity for the vascular endothelial cells. The coating layer contains, for example, a coating agent with an affinity for the blood endothelial cells, and examples of the coating agent include agents such as those described later. Meanwhile, in the organ-on-a-chip 100, the inner walls of the second channel 20 and the third channels 30 may have no affinity for the vascular endothelial cells for the purpose of, for example, suppressing fixing of the vascular endothelial cells even if the vascular endothelial cells introduced to the first channel 10 to form the blood vessel model also flow into the second channel 20 through the third channels 30. Specifically, it is preferable that, for example, the inner walls do not have a coating layer with an affinity for the vascular endothelial cells.

The second channel 20 is an introduction channel for introducing a sample containing a cell mass from the upstream side toward the downstream side, and includes a trap portion 22. The trap portion 22 is formed between an upstream region 21 and a downstream region 23 of the second channel 20. There is no particular limitation on the shape of the trap portion 22 as long as the trap portion 22 can trap the cell mass when the sample is introduced from the upstream side toward the downstream side in the second channel 20.

It is preferable that the trap portion 22 allows single cells to pass through for the reason that, for example, the trap portion 22 traps the cell mass but does not trap vascular endothelial cells that flow thereinto from the first channel 10 through the third channels 30 or unwanted cells. It is preferable that the trap portion 22 has a shape and a size as described below.

As an example of the shape of the trap portion 22, in the second channel 20, the cross section of the inner space of the trap portion 22 is made smaller (narrower) than that of the upstream region 21, as shown in FIG. 1. Specifically, it is preferable that in the second channel 20, for example, the cross-sectional area of the inner space of the trap portion 22 is smaller than the cross-sectional area of the cross section of the cell mass. The above-mentioned cross section of the cell mass is, for example, a cross section of the cell mass passing through the center of the cell mass, and it is preferable that the cross-sectional area of the inner space of the trap portion 22 is smaller than, for example, the minimum cross-sectional area of the cross sections of the cell mass passing through the center of the cell mass. In addition, it is preferable that the cross-sectional area of the inner space of the trap portion 22 is larger than the maximum cross-sectional area of cross sections of a single cell. The above-mentioned cross section of the cell is, for example, a cross section having the maximum cross-sectional area, and may or may not pass through the center of the cell. It is preferable that the cross-sectional area of the inner space of the trap portion 22 is, for example, smaller than the minimum cross-sectional area of cross sections of the cell mass passing through the center of the cell mass and larger than the maximum cross-sectional area of cross sections of a single cell. Moreover, in the second channel 20, the upstream region 21 need only have such a size that allows the cell mass introduced from the inlet 421 to move to the trap portion 22 on the downstream side, and specifically, it is preferable that, for example, the cross-sectional area of the inner space of the upstream region 21 is larger than the maximum cross-sectional area of the cell mass. In the second channel 20, there is no particular limitation on the cross-sectional area of the inner space of the downstream region 23. For example, the cross-sectional area of the inner space of the downstream region 23 may be the same as the cross-sectional area of the inner space of the trap portion 22 or the cross-sectional area of the inner space of the upstream region 21.

In a specific example, in terms of the shape of the second channel 20, the inner space has, for example, a circular cross section in a direction perpendicular to the axial direction of the second channel 20. In this case, in the second channel 20, a cross section of the trap portion 22 has, for example, a diameter of 10 to 300 pm, 50 to 250 pm, or 100 to 200 pm, a cross section of the upstream region 21 has, for example, a diameter of 200 to 1000 pm, 300 to 800 pm, or 400 to 700 pm, and the diameter of the trap portion 22 is, for example, 0.1 to 0.9 times as large as the diameter of the upstream region 21. The circular shape may be, for example, a perfect circular shape or an elliptic shape. Also, in terms of the shape of the second channel 20, the inner space may have, for example, a quadrilateral cross section in a direction perpendicular to the axial direction of the second channel 20. In this case, in the second channel 20, a cross section of the trap portion 22 has, for example, a width (in the X direction in FIG. 1) and a height (in the Z direction in FIG. 1) of 10 to 300 pm, 50 to 250 pm, or 100 to 200 pm, a cross section of the upstream region 21 has, for example, a width and a height of 200 to 1000 pm, 300 to 800 pm, or 400 to 700 pm, and the width and the height of the trap portion 22 are, for example, 0.1 to 0.9 times as large as the width and the height of the upstream region 21. The quadrilateral cross section may have, for example, a square shape or a rectangular shape. Moreover, the cross section may have a polygonal shape other than a quadrilateral shape.

There is no particular limitation on the length of the second channel 20 in the axial direction. For example, the length from the upstream end to the downstream end is 0.5 to 80 mm, the length from the upstream end of the second channel 20 to the upstream end of the trap portion 22 is 0.25 to 40 mm, the length of the trap portion 22 is 10 to 100 pm, and the length from the downstream end of the trap portion 22 to the downstream end of the second channel 20 is 0.25 to 40 mm.

The shape of the trap portion 22 is not limited to this example, and for example, in the second channel 20, the cross-sectional area of the inner space may be larger than the minimum cross-sectional area of cross sections of the cell mass. Exemplary conditions for such an example are as follows. In a direction perpendicular to the axial direction of the second channel 20, the inner space of the trap portion 22 has, for example, a quadrilateral cross section, and the lengths of at least a pair of sides of two pairs of opposite sides of the cross section are preferably smaller than the diameter of the cell mass, and more preferably smaller than the minimum diameter of the cell mass. In this case, for example, irrespective of the cross-sectional area of the inner space as described above, that is, even when the cross-sectional area of the inner space is smaller than the minimum cross-sectional area of the cell mass, the trap portion 22 can trap the cell mass. In the case where the inner space of the trap portion 22 has a circular cross section, it is preferable that the minimum diameter of the cross section is smaller than the minimum diameter of the cell mass, for example.

The third channel group includes a plurality of third channels 30, and the third channels 30 are channels that communicate between the first channel 10 and the second channel 20.

The third channels 30 of the organ-on-a-chip 100 are channels that communicate between the first channel 10 and the second channel 20 to enable interaction between a blood vessel model formed in the first channel 10 and a cell mass introduced to the second channel 20 when the organ-on-a-chip 100 is used. Accordingly, it is preferable that the sizes of cross sections of the third channels 30 in a direction perpendicular to the axial direction of the third channels 30 are determined such that, for example, a substance released from the cell mass, a substance or a cell released from the blood vessel model, a cell (e.g., immunocyte) passing through the blood vessel model, a sprout extending from the blood vessel model, and the like can pass through the third channels 30, and the cell mass cannot pass through the third channels 30.

In the organ-on-a-chip 100, coupling holes are formed at portions where the first channel 10 is coupled to the third channels 30. The cross sections of the inner spaces of the coupling holes correspond to the cross sections of the inner spaces of the third channels 30, and preferably have a size that allows a tubular blood vessel model to be formed covering the coupling holes inside the first channel 10.

From this viewpoint, in terms of the shape of the third channels 30, the inner spaces have, for example, a circular cross section in a direction perpendicular to the axial direction of the third channels 30, and the cross section has, for example, a diameter of 10 to 100 pm, 20 to 90 pm, or 30 to 80 pm. The circular shape may be, for example, a perfect circular shape or an elliptic shape. Also, in terms of the shape of the third channels 30, the inner space may have, for example, a quadrilateral cross section in a direction perpendicular to the axial direction of the third channels 30, and the cross section has, for example, a width (in the Y direction in FIG. 1) and a height (in the Z direction in FIG. 1) of 10 to 100 pm, 20 to 90 pm, or 30 to 80 pm. The quadrilateral cross section may have, for example, a square shape or a rectangular shape. Moreover, the cross section may have a polygonal shape other than a quadrilateral shape.

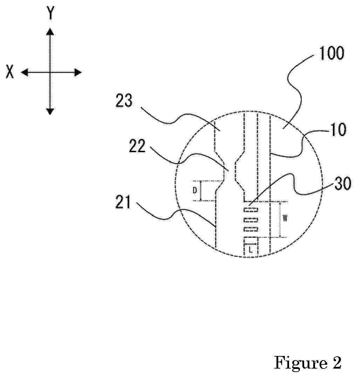

A partially enlarged view illustrating a region of the third channel group (which includes a plurality of third channels 30) in the organ-on-a-chip 100 in FIG. 1 is shown in FIG. 2. FIG. 2 is a partial top view of the organ-on-a-chip 100. A length L (in the X direction in FIG. 2) of the third channels 30 is, for example, 10 to 500 pm, 50 to 300 pm, or 100 to 200 pm.

The number of the third channels 30 included in the third channel group is not particularly limited, and is, for example, 2 to 500, 2 to 300, or 2 to 100. The length of a region where the third channel group couples the first channel 10 and the second channel 20, that is, a distant W (in the Y direction in FIG. 2) from the third channel 30 on the upstream side to the third channel 30 on the downstream side, is, for example, 30 to 10000 pm, 30 to 5000 pm, or 30 to 1000 pm. The pitch between the third channels 30, that is, a distance (in the Y direction in FIG. 2) between the adjacent third channels 30, is, for example, 5 to 100 pm, 10 to 50 pm, or 15 to 30 pm.

There is no particular limitation on the position of the third channel group, and a distance between the third channel 30 on the downstream side of the third channel group and an end on the upstream side of the trap portion 22 in the second channel 20 is, for example, 0 to 1000 pm, 0 to 500 pm, or 0 to 200 pm. Regarding how a living organism is reproduced in the organ-on-a-chip 100, for example, a substance released from the cell mass is detected, via the third channels 30, by a blood vessel model formed in the first channel 10. Accordingly, it is preferable that a portion of the second channel 20 to which the third channel group is coupled is located, for example, upstream of the trap portion 22 in the second channel 20 as shown in FIGS. 1 and 2. Specifically, it is preferable that the third channel group is disposed, for example, at a position that is a certain distance away from an end on the upstream side of the trap portion 22 in the second channel 20, and the certain distance (D in FIG. 2) is, for example, larger than the diameter of the cell mass. The distance is, for example, more than 0 pm and 1000 pm or less, more than 0 pm and 500 pm or less, or more than 0 pm and 200 pm or less.

Next, a method of using the organ-on-a-chip 100 of Embodiment 1 (biological function reproduction method) will be described.

First, as first culturing, a blood vessel model is formed on the inner wall of the first channel 10 by introducing the vascular endothelial cells to the first channel 10 through the inlet 411 of the organ-on-a-chip 100 and culturing the cells. It is preferable to introduce the vascular endothelial cells and a culture medium together, for example, as a cell-containing liquid. There is no particular limitation on the type of vascular endothelial cell, and examples thereof include umbilical vein endothelial cells such as HUVEC cells. The culture medium is not particularly limited and can be selected, for example, depending on the type of vascular cell. Specific examples thereof include EBM2 and the like.

The introduction conditions and the culture conditions are not particularly limited, and are as follows, for example. That is to say, a suspension containing HUVEC cells at a concentration of 10^{×6} cells/mL is introduced through the inlet 411 of the organ-on-a-chip 100 at 37°C until the first channel 10 is filled with the cell suspension, and then the cell suspension is left to stand for 4 hours to fix the HUVEC cells on the inner surface of the first channel 10. Thereafter, the cells are cultured with a culture medium (EBM2) delivered through the inlet 411 at 1000 pL/minute for 12 hours using a syringe pump. With such first culturing, a HUVEC-cell tube (blood vessel model) with a thickness of about 20 pm can be formed on the entire inner circumferential surface of the first channel 10, for example.

Next, as second culturing, a sample containing the cell mass is introduced through the inlet 421 of the organ-on-a-chip 100 and is cultured. There is no particular limitation on the number of cell masses included in the sample. For example, one cell mass per organ-on-a-chip 100 is preferable because it is important to examine a phenomenon caused by a single cell mass. It is preferable that the sample contains, for example, the cell mass and a culture medium. There is no particular limitation on the type of culture medium, and examples thereof include EBM2 and the like.

The type of cell mass is not particularly limited, and is preferably a cell mass of cancer cells, for example. The size of the cell mass is, for example, 100 to 1000 pm, 200 to 800 pm, or 300 to 500 pm.

The introduction conditions and the culture conditions are not particularly limited, and are as follows, for example. That is to say, for example, after a sample containing a single cell mass is introduced to the inlet 421 of the organ-on-a-chip 100 using a pipette chip at 37°C, the sample liquid is delivered from the inlet 421 toward the outlet 422 of the second channel 20 using a syringe pump.

In the second culturing, while the sample flows from the inlet 421 toward the outlet 422, the cell mass arrives at the trap portion 22 and is trapped in the trap portion 22. Then, when the blood vessel model formed in the first channel 10 detects, through the third channels 30, a substance released from the cell mass trapped and cultured in the trap portion 22 in the second channel 20, sprouts appear from the blood vessel model, and then the sprouts can grow inside the third channels 30 toward the second channel 20 via portions (coupling holes) where the third channels 30 are coupled to the first channel 10. Thus, using the organ-on-a-chip 100 of this embodiment makes it possible to reproduce a biological process between a cell mass and a blood vessel.

### (Embodiment 2)

In an organ-on-a-chip of this embodiment, the trap portion in the first channel includes columnar bodies (pillars) that prevent the cell mass from moving toward the downstream side. The organ-on-a-chip of this embodiment is the same as the organ-on-a-chip of Embodiment 1, except that the trap portion is changed. FIGS. 1 and 2 described above can be applied to the basic structure of the organ-on-a-chip of this embodiment.

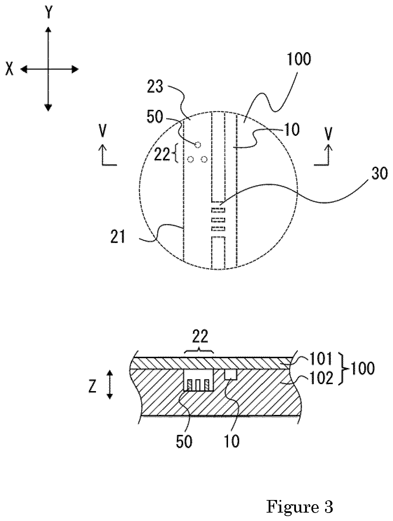

As an example, partially enlarged views illustrating a region that includes a trap portion in the first channel of the organ-on-a-chip are shown in FIG. 3. Note that, in FIG. 3, the upper diagram is a partial top view of the organ-on-a-chip 100 as in FIG. 2 described above, and the lower diagram is a cross-sectional view taken along line V-V

As shown in FIG. 3, three columnar bodies 50 are disposed inside the second channel 20, and the region in which the columnar bodies 50 are disposed serves as the trap portion 22. With this embodiment, the columnar bodies 50 trap the cell mass, and therefore, in the second channel 20, the size of the cross-sectional area of the inner space of the trap portion 20 may be the same as that of the cross-sectional area of the inner space of the upstream region 21, for example, unlike Embodiment 1 above.

The number and the size of the columnar bodies 50 in the trap portion 22 are not particularly limited, and can be determined as appropriate depending on the size of a cell mass to be trapped, the size of the cross section of the inner space of the trap portion 22, and the like. The number of the columnar bodies 50 is, for example, 1 to 10, 2 to 8, or 3 to 5.

The shape of each columnar body 50 is not particularly limited, and may be a cylindrical shape, for example, as shown in FIG. 3, or a prismatic shape. It is preferable that the area of contact between the cell mass and the columnar body 50 is small, for example, because conditions close to the in-vivo conditions can be achieved. In terms of the size of each columnar body 50, for example, the height in the Z direction is 10 to 1000 pm or 50 to 500 pm, the width in the X direction is 5 to 200 pm or 10 to 100 pm, and the width in the Y direction is 5 to 200 pm or 10 to 100 pm. It is preferable that the columnar body 50 is, for example, shorter than or equal to half of the height of the inner space of the second channel 20. Also, it is preferable that the height of the columnar body 50 is, for example, 0.1 times or more as long as the diameter of the cell mass.

There is no particular limitation on the positions of the columnar bodies 50 in the trap portion 22, and it is preferable that, for example, in a direction (e.g., the X direction in FIG. 3) perpendicular to the axial direction of the second channel 20, the columnar bodies 50 are disposed on the inner wall on a side opposite to portions that communicate with the third channels 30. Thus, the trap portion 22 in the second channel 20 can trap the cell mass at a position away from the portions that communicate with the third channels 30. Regarding how the organ-on-a-chip is used, as described above, for example, a substance released from the cell mass is detected, via the third channels 30, by a blood vessel model formed in the first channel 10. At this time, securing a distance from the cell mass to the third channels 30 makes it possible to form a gradation of the concentration of the released substance in the region therebetween and achieve a state closer to the in-vivo state.

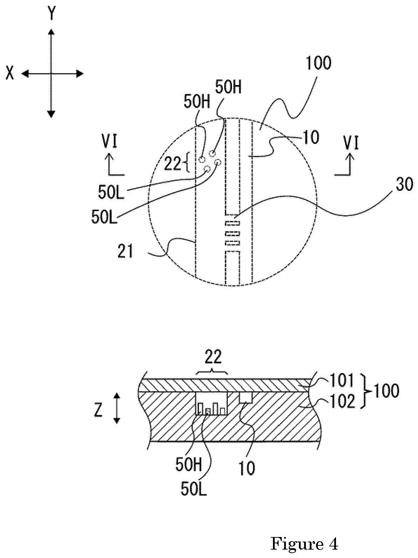

As another example of the trap portion 22 in which the columnar bodies 50 are disposed, partially enlarged views illustrating a region that includes a trap portion in the first channel of the organ-on-a-chip are shown in FIG. 4. Note that, in FIG. 4, the upper diagram is a partial top view of the organ-on-a-chip 100, and the lower diagram is a cross-sectional view taken along line VI-VI. The description of FIG. 3 can be applied to this example unless otherwise stated.

As shown in FIG. 4, four columnar bodies 50 are disposed inside the second channel 20, and the region in which the columnar bodies 50 are disposed serves as the trap portion 22. The four columnar bodies 50 include two columnar bodies 50H that have a relatively large height in the Z direction, and two columnar bodies 50L that have relatively low height in the Z direction. In this aspect, the two columnar bodies 50H are the same as the columnar bodies 50 in FIG. 3 described above and can trap the cell mass. The columnar bodies 50H may also be referred to as "trapping columnar bodies". Meanwhile, the two columnar bodies 50L with a lower height may also be referred to as "basal columnar bodies" for preventing the cell mass from coming into contact with the inner wall of the second channel 20. That is to say, as described above, it is preferable that a state in the organ-on-a-chip is closer to the in-vivo state, and therefore, it is desired that, when the trap portion 22 traps the cell mass, the cell mass floats in the inner space of the second channel 20, that is, the cell mass is not in contact with the inner wall. Accordingly, due to the basal columnar bodies 50L with a relatively low height being disposed, a cell mass that flows into the trap portion 22 in the second channel 20 gets on tops of the basal columnar bodies 50L, and the trapping columnar bodies 50H can trap the cell mass so as to prevent the cell mass from moving toward the downstream side therefrom. With this aspect, an obstacle to the formation of a gradation of a substance released from the cell mass as described above is further reduced, thus making it possible to achieve a state even closer to the in-vivo state.

The numbers and the sizes of the columnar bodies 50H and 50L in the trap portion 22 are not particularly limited, and can be determined as appropriate depending on the size of a cell mass to be trapped, the size of the cross section of the inner space of the trap portion 22, and the like. It is preferable that the area of contact between the cell mass and each of the columnar bodies 50H and 50L is small, for example, because conditions close to the in-vivo conditions can be achieved. The number of the trapping columnar bodies 50H is, for example, 1 to 10, 2 to 8, or 3 to 5, and the number of the basal columnar bodies 50L is, for example, 1 to 10, 1 to 5, or 1 to 3.

In a specific example, the shape and the size of each trapping columnar body 50H are not particularly limited, and are, for example, the same as those in the description of FIG. 3. The shape of each basal columnar body 50L may be, for example, a cylindrical shape or a prismatic shape. In terms of the size of each columnar body 50L, for example, the height in the Z direction is 5 to 100 pm or 10 to 50 pm, the width in the X direction is 5 to 200 pm or 10 to 100 pm, and the width in the Y direction is 5 to 200 pm or 10 to 100 pm.

There is no particular limitation on the positions of the columnar bodies 50 in a direction perpendicular to the axial direction of the second channel 20, and it is preferable that, for example, the columnar bodies 50 are disposed at positions away from a portion of the second channel 20 that communicates with the third channel group. Specifically, it is preferable that the columnar bodies 50 are disposed at positions 100 to 1000 pm away from a portion of the second channel 20 that communicates with the third channel group.

### (Embodiment 3)

In the examples of the organs-on-a-chip shown in the embodiments described above, the inner spaces of the first channel, the second channel, the third channels, and the like are formed by the recessed portions on the opposed surface of the lower substrate and the opposed surfaces of the upper substrate that covers the recessed portions. The organ-on-a-chip is not limited to these aspects, and an aspect may also be employed in which, for example, recessed portions are formed on both the opposed surface of the lower substrate and the opposed surface of the upper substrate and these substrates are laminated.

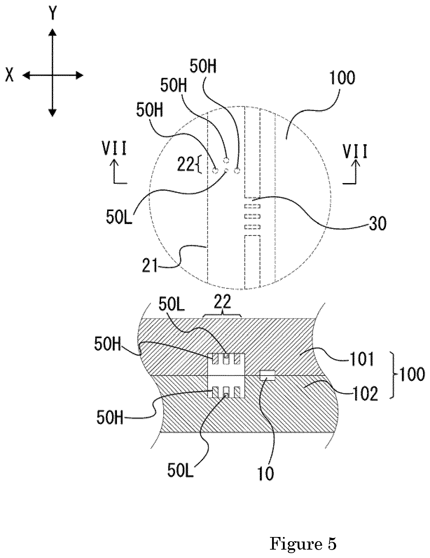

That is to say, for example, an organ-on-a-chip of this embodiment includes a pair of substrates (an upper substrate and a lower substrate), opposed surfaces of one of the substrates and the other substrate are each provided with recessed portions serving as inner spaces of the first channel, the second channel, and the third channel group, and the recessed portions on the opposed surface of the first substrate and the recessed portions on the opposed surface of the second substrate form the first channel, the second channel, and the third channel group. FIG. 5 shows an example of an organ-on-a-chip of this embodiment. Note that, in FIG. 5, the upper diagram is a partial top view of the organ-on-a-chip 100, and the lower diagram is a cross-sectional view taken along line VII-VII. The description of FIG. 4 can be applied to this example unless otherwise stated.

As shown in FIG. 5, the inner space of the trap portion 22 in the second channel 20 is formed by the recessed portion on the upper substrate 101 and the recessed portion on the lower substrate 102. In the trap portion 22, on the bottom surface of the recessed portion on the lower substrate 102 and the bottom surface of the recessed portion on the upper substrate 101, the trapping columnar bodies 50H are disposed at corresponding three positions (apexes of a triangle) so that the trapping columnar bodies 50H on the lower substrate and the trapping columnar bodies 50H on the upper substrate make a pair, and the basal columnar bodies 50L are disposed at positions corresponding to the center of the base of the above-mentioned triangle such that the basal columnar body 50L on the lower substrate and the basal columnar body 50L on the upper substrate make a pair. Accordingly, with the organ-on-a-chip of this embodiment, the trapping columnar bodies 50H and the basal columnar bodies 50L are disposed on both the upper side and the lower side in the inner space of the trap portion 22, thus making it possible to achieve conditions close to the in-vivo conditions as in FIG. 4 described in Embodiment 2.

Furthermore, as shown in FIG. 5, in the organ-on-a-chip of this embodiment, the first channel 10 and the second channel 20 communicate with each other via the third channels 30 located at the center in the height direction of the first channel 10 and the center in the height direction of the second channel 20. From this viewpoint as well, it is possible to, for example, form a gradation of a substance released from the cell mass and achieve conditions even closer to the in-vivo conditions.

### (Embodiment 4)

For example, the biological function reproduction method of the present invention further includes performing coating for forming a coating layer with an affinity for vascular endothelial cells on an inner wall of the first channel prior to the first culturing for forming a blood vessel model in the first channel of the organ-on-a-chip of the present invention. Hereinafter, this example will be described with reference to FIG. 1 described above, but the present invention is not limited to this example.

(4-1)
In the coating, for example, a coating liquid that contains a coating agent for fixing the vascular endothelial cells and a solvent that does not contain the coating agent are introduced to the first channel 10 and the second channel 20, respectively, with a flow rate balance that prevents flow in the third channels 30. This adjustment makes it possible to introduce the coating liquid to only the first channel 10 to form a coating layer with an affinity for the vascular endothelial cells in only the first channel 10. With this aspect, it is possible to more efficiently fix the vascular endothelial cells introduced to the first channel 10 on the inner wall of the first channel 10.

Examples of the coating agent include collagens such as Collagen I. The coating liquid contains, for example the coating agent and a solvent, and examples of the solvent include buffer solutions and buffered saline solutions such as PBS. In the coating, there is no particular limitation on the amount of the coating agent used, the treatment conditions such as a temperature and a period of time, and the like, and for example, 100 pL of the coating liquid is used and incubated at 25°C for 1 hour.

In this aspect, it is preferable that, in the following first culturing, for example, a culture medium that contains the vascular endothelial cells and a solvent that does not contain the cells are introduced to the first channel 10 and the second channel 20, respectively, with a flow rate balance that prevents flow in the third channels 30. This adjustment makes it possible to prevent the vascular endothelial cells introduced to the first channel 10 from flowing into the second channel 20 through the third channels 30. Note that the coating layer is formed only in the first channel 10 and is not formed in the second channel 20 in the coating, and therefore, even if the vascular endothelial cells flow into the second channel 20, the vascular endothelial cells are less likely to be fixed to the second channel 20 compared with the first channel 10 and are likely to be discharged to the outlet 422. There is no particular limitation on the solvent introduced to the second channel 20, and examples thereof include culture mediums, buffer solutions, and buffered saline solutions such as PBS.

Conditions where the coating liquid and the solvent are introduced to the first channel 10 and the second channel 20, respectively, in the coating with a flow rate balance that prevents flow in the third channels 30 can be determined as follows, for example. That is to say, for example, the flow rate in the first channel 10 and the flow rate in the second channel 20 are determined such that the ratio between the flow rates is the same as the ratio between the cross-sectional area of the first channel 10 and the cross-sectional area of the second channel 20, and the coating liquid and the solvent are delivered at thus determined flow rates using syringe pumps. Note that conditions where the culture medium that contains the vascular endothelial cells and the solvent that does not contain the cells are introduced to the first channel 10 and the second channel 20, respectively, in the first culturing with a flow rate balance that prevents flow in the third channels 30 can be determined in the same manner.

(4-2)
After the coating in (4-1) above, for example, the first culturing may also include introducing a culture medium containing the vascular endothelial cells to the overall channel that includes the first channel 10, the second channel 20, and the third channels 30. As described above, the coating layer is formed only in the first channel 10 and is not formed in the second channel 20 in the coating, and therefore, the vascular endothelial cells are less likely to be fixed to the second channel 20 compared with the first channel 10 and are likely to be discharged to the outlet 422.

Note that the coating layer is formed only in the first channel 10 and is not formed in the second channel 20 in the coating, and therefore, even if the vascular endothelial cells flow into the second channel 20, the vascular endothelial cells are less likely to be fixed to the second channel 20 compared with the first channel 10 and are likely to be discharged to the outlet.

As described above, the present invention has been described with reference to the embodiments, but the present invention is not limited to the above-described embodiments. Various modifications that can be understood by a person skilled in the art can be made in the configurations and details of the present invention without departing from the scope of the present invention.

### Industrial Applicability

As described above, with the present invention, the second channel includes the trap portion, and therefore, the cell mass can be trapped in the trap portion when being introduced as a sample, and thus it can be said that conditions closer to the in-vivo conditions can be reproduced.

### Description of Reference Numerals

- 100: Organ-on-a-chip
- 101: Upper substrate
- 102: Lower substrate
- 10: First channel
- 20: Second channel
- 21: Upstream region
- 22: Trap portion
- 23: Downstream region
- 30: Third channel
- 411, 421: Inlet
- 412, 422: Outlet
- 50: Columnar body
- 50H: Trapping columnar body
- 50L: Basal columnar body

## Claims

1. An organ-on-a-chip for reproducing a biological process between a cell mass and a blood vessel, comprising:
a first channel;
a second channel; and
a third channel group,
wherein the first channel is an introduction channel for introducing vascular endothelial cells for forming a blood vessel model on an inner wall of the first channel through cell culture,
the second channel is an introduction channel for introducing a sample containing a cell mass from an upstream side toward a downstream side, and includes a trap portion for trapping the cell mass and located between the upstream side and the downstream side inside the second channel, and
the third channel group includes a plurality of third channels, and the third channels are channels that communicate between the first channel and the second channel.

2. The organ-on-a-chip according to claim 1,
wherein the trap portion in the second channel traps the cell mass and allows single cells to pass through.

3. The organ-on-a-chip according to claim 1 or 2,
wherein, in a direction perpendicular to an axial direction of the second channel,
a cross-sectional area of an inner space of the trap portion is smaller than the minimum cross-sectional area of cross sections of the cell mass passing through the center of the cell mass.

4. The organ-on-a-chip according to claim 3,
wherein, in a direction perpendicular to the axial direction of the second channel,
the cross-sectional area of the inner space of the trap portion is larger than the maximum cross-sectional area of cross sections of single cells.

5. The organ-on-a-chip according to any one of claims 1 to 4,
wherein, in a direction perpendicular to an axial direction of the second channel,
when an inner space has a circular cross section, a cross section of the trap portion has a diameter of 10 to 300 pm, a cross section of a portion located upstream of the trap portion has a diameter of 200 to 1000 pm, and the diameter of the trap portion is 0.1 to 0.9 times as large as the diameter of the portion located upstream of the trap portion, and
when an inner space has a quadrilateral cross section, a cross section of the trap portion has a width and a height of 10 to 300 pm, a cross section of a portion located upstream of the trap portion has a width and a height of 200 to 1000 pm, and the width and the height of the trap portion are 0.1 to 0.9 times as large as the width and the height of the portion located upstream of the trap portion.

6. The organ-on-a-chip according to any one of claims 1 to 5,
wherein, in a direction perpendicular to an axial direction of the second channel,
an inner space of the trap portion has a quadrilateral cross section, and
lengths of at least a pair of sides of two pairs of opposite sides of the cross section are smaller than a diameter of the cell mass.

7. The organ-on-a-chip according to any one of claims 1 to 6,
wherein, in a direction perpendicular to an axial direction of the second channel,
the trap portion in the second channel has a columnar body that prevents the cell mass from moving toward the downstream side.

8. The organ-on-a-chip according to claim 7,
wherein, in a direction perpendicular to the axial direction of the second channel,
a height of the columnar body of the trap portion is shorter than a height of an inner space of the trap portion.

9. The organ-on-a-chip according to claim 8,
wherein the height of the columnar body of the trap portion is shorter than or equal to half of the height of the inner space of the trap portion.

10. The organ-on-a-chip according to any one of claims 7 to 9,
wherein the columnar body is disposed at a position 100 to 1000 pm away from a portion of the second channel that communicates with the third channel group.

11. The organ-on-a-chip according to any one of claims 1 to 10,
wherein the third channel group is disposed upstream of the trap portion in the second channel.

12. The organ-on-a-chip according to claim 11,
wherein the third channel group is disposed at a position 0 to 1000 pm away from an end on an upstream side of the trap portion in the second channel.

13. The organ-on-a-chip according to any one of claims 1 to 12,
wherein, in a direction perpendicular to an axial direction of the first channel,
a length of an inner circumference of the first channel corresponds to a length of an outer circumference of a blood vessel.

14. The organ-on-a-chip according to any one of claims 1 to 13,
wherein, in a direction perpendicular to an axial direction of the first channel,
when an inner space of the first channel has a circular cross section, the cross section has a diameter of 100 to 500 pm, and
when an inner space of the first channel has a quadrilateral cross section, the cross section has a width and a height of 100 to 500 pm.

15. The organ-on-a-chip according to any one of claims 1 to 14,
wherein the inner wall of the first channel has a coating layer with an affinity for vascular endothelial cells.

16. The organ-on-a-chip according to any one of claims 1 to 15,
wherein, when an inner space of each of the third channels has a circular cross section in a direction perpendicular to an axial direction of the third channel, the cross section has a diameter of 10 to 100 pm, and
when an inner space of each of the third channels has a quadrilateral cross section in a direction perpendicular to the axial direction of the third channel, the cross section has a width and a height of 10 to 100 pm.

17. The organ-on-a-chip according to any one of claims 1 to 16, comprising
a pair of substrates,
wherein one of the substrates is placed on the other substrate,
an opposed surface of the one substrate is provided with recessed portions serving as inner spaces of the first channel, the second channel, and the third channel group, and
the recessed portions on the opposed surface of the one substrate and an opposed surface of the other substrate form the first channel, the second channel, and the third channel group.

18. The organ-on-a-chip according to any one of claims 1 to 16, comprising
a pair of substrates,
wherein opposed surfaces of one of the substrates and the other substrate are each provided with recessed portions serving as inner spaces of the first channel, the second channel, and the third channel group, and
the recessed portions on the opposed surface of the first substrate and the recessed portions on the opposed surface of the second substrate form the first channel, the second channel, and the third channel group.

19. A biological function reproduction method for causing a cell mass and a blood vessel model interact with each other, the method using the organ-ona-chip according to any one of claims 1 to 18, and comprising:
performing first culturing for forming a blood vessel model on an inner wall of the first channel by introducing vascular endothelial cells to the first channel and culturing the cells; and
performing second culturing for introducing a sample containing a cell mass to the second channel from an upstream side of the second channel and culturing the cell mass in a state in which the cell mass is trapped in the trap portion in the second channel.

20. The biological function reproduction method according to claim 19,
wherein the vascular endothelial cells are HUVEC cells.

21. The biological function reproduction method according to claim 19 or 20,
wherein the vascular endothelial cells are introduced together with a culture medium in the first culturing.

22. The biological function reproduction method according to any one of claims 19 to 21,
wherein the cell mass is a cell mass of cancer cells.

23. The biological function reproduction method according to any one of claims 19 to 22,
wherein the sample contains the cell mass and a culture medium in the second culturing.

24. The biological function reproduction method according to any one of claims 19 to 23, further comprising
performing coating for forming a coating layer with an affinity for the vascular endothelial cells on the inner wall of the first channel prior to the first culturing.

25. The biological function reproduction method according to claim 26,
wherein, in the coating, a coating liquid that contains a coating agent for fixing the vascular endothelial cells and a solvent that does not contain the coating agent are introduced to the first channel and the second channel, respectively, with a flow rate balance that prevents flow in the third channels, and
in the first culturing, a culture medium that contains the vascular endothelial cells and a solvent that does not contain the cells are introduced to the first channel and the second channel, respectively, with a flow rate balance that prevents flow in the third channels.
